(19) Europäisches Patentamt
European Patent Office
Office européen des brevets

(11) Veröffentlichungsnummer: **0 413 405 A2**

(12) **EUROPÄISCHE PATENTANMELDUNG**

(21) Anmeldenummer: **90250206.1**

(22) Anmeldetag: **14.08.90**

(51) Int. Cl.$^5$: **C07C 231/04**, A61K 49/00

(30) Priorität: **16.08.89 DE 3927444**

(43) Veröffentlichungstag der Anmeldung: **20.02.91 Patentblatt 91/08**

(84) Benannte Vertragsstaaten: **AT BE CH DE DK ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Radüchel, Bernd, Dr.**
**Gollanczstrasse 132**
**D-1000 Berlin 28(DE)**
Erfinder: **Schmitt-Willich, Heribert, Dr.**
**Gardeschützenweg 102**
**D-1000 Berlin 45(DE)**
Erfinder: **Gries, Heinz, Dr.**
**Helmstedter Strasse 19**
**D-1000 Berlin 31(DE)**
Erfinder: **Schuhmann-Giampieri, Gabriele, Dr.**
**Marschnerstrasse 34**
**D-1000 Berlin 45(DE)**
Erfinder: **Vogler, Hubert, Dr.**
**Dahlmannstrasse 10**
**D-1000 Berlin 12(DE)**

(54) **Verwendung von Amid-Komplexverbindungen.**

(57) Komplexverbindungen der allgemeinen Formel I

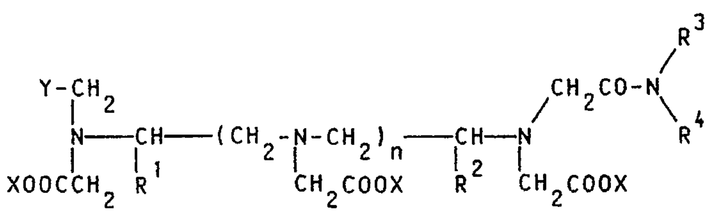

(I)

worin
n die Ziffern 0, 1 oder 2,
$R^1$ und $R^2$ Wasserstoffatome, niedere Alkylgruppen, Phenylgruppen, Benzylgruppen oder, wenn n für die Ziffer 0 steht, auch gemeinsam eine Trimethylen- oder eine Tetramethylengruppe,
$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, wenigstens eine $R^3$-Gruppe, eine Cycloalkyl- oder eine gegebenenfalls durch eine oder mehrere Di-$C_1$-$C_6$-alkanylamino- oder durch eine oder mehrere $C_1$-$C_6$-Alkoxygruppen substituierte Aryl- oder Aralkylgruppe,
$R^4$ ein Wasserstoffatom, ein gesättigter, ungesättigter, gerad- oder verzweigtkettiger oder cyclischer Kohlenwasserstoffrest mit bis zu 16 C-Atomen, oder
$R^3$ und $R^4$ gemeinsam einen gesättigten oder ungesättigten, gegebenenfalls durch einen oder mehrere $C_1$-$C_6$-Alkyl-, $C_1$-$C_5$-Hydroxyalkyl-, einen gegebenenfalls hydroxylierten oder $C_1$-$C_6$-alkoxylierten $C_2$-$C_6$-Acyl-, Hydroxy-, Carbamoyl-, Carbamoyl-substituierten $C_1$-$C_6$-Alkyl-, am Carbamoyl-Stickstoff durch einen oder zwei $C_1$-$C_6$-Alkylrest(e) - die auch einen gegebenenfalls ein Sauerstoffatom enthaltenen Ring bilden können - substituierten Carbamoyl- oder einen $C_1$-$C_6$-Acylamino- oder $C_1$-$C_6$-Alkylaminorest substituierten 5- oder 6-Ring, der gegebenenfalls ein weiteres Stickstoff-, Sauerstoff-, Schwefelatom oder eine Carbonylgruppe enthält.

EP 0 413 405 A2

X ein Wasserstoffatom und/oder ein Metallionenäquivalent, mindestens eines Elements der Ordungszahlen 21-29, 42, 44, oder 58-70,
Y eine

COOX- oder 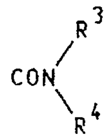 -Gruppe

bedeuten, mit der Maßgabe, daß mindestens zwei der Substituenten X für ein Metallionenäquivalent stehen, sowie deren Salze mit organischen und/oder anorganischen Basen, sind wertvolle Verbindungen zur organspezifischen NMR-Diagnostik und bei Patienten mit Niereninsuffizienz.

# VERWENDUNG VON AMID-KOMPLEXVERBINDUNGEN

Die vorliegende Erfindung betrifft den in den Patentansprüchen gekennzeichneten Gegenstand, das heißt die Verwendung physiologisch vertraglicher Amidkomplexverbindungen für die Herstellung von Mitteln für die organspezifische NMR-Diagnostik und bei Patienten mit Niereninsuffizienz sowie die Anwendung dieser Verbindungen für den angegebenen Zweck.

In der europäischen Patentanmeldung Publikationsnummer 263059 werden Verbindungen der allgemeinen Formel I

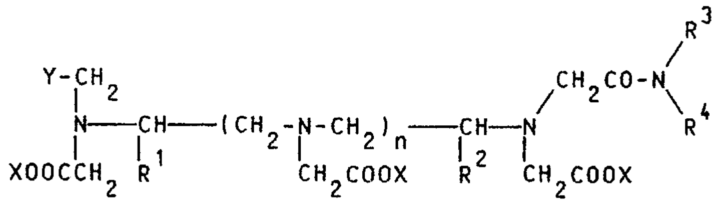

(I)

beansprucht, worin

n die Ziffern 0, 1 oder 2,

$R^1$ und $R^2$ Wasserstoffatome, niedere Alkylgruppen, Phenylgruppen, Benzylgruppen oder, wenn n für die Ziffer 0 steht, auch gemeinsam eine Trimethylen- oder eine Tetramethylengruppe,

$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, wenigstens eine $R^3$-Gruppe, eine Cycloalkyl-oder eine gegebenenfalls durch eine oder mehrere Di-$C_1$-$C_6$-alkylamino- oder durch eine oder mehrere $C_1$-$C_6$-Alkoxygruppen substituierte Aryl- oder Aralkylgruppe,

$R^4$ ein Wasserstoffatom, ein gesättigter, ungesättigter, gerad- oder verzweigtkettiger oder cyclischer Kohlenwasserstoffrest mit bis zu 16 C-Atomen, oder

$R^3$ und $R^4$ gemeinsam einen gesättigten oder ungesättigten, gegebenenfalls durch einen oder mehrere $C_1$-$C_6$-Alkyl-, $C_1$-$C_5$-Hydroxyalkyl-, einen gegebenenfalls hydroxylierten oder $C_1$-$C_6$-alkoxylierten $C_2$-$C_6$-Acyl-, Hydroxy-, Carbamoyl-, Carbamoyl-substituierten $C_1$-$C_6$-Alkyl-, am Carbamoyl-Stickstoff durch einen oder zwei $C_1$-$C_6$-Alkylrest(e) - die auch einen gegebenenfalls ein Sauerstoffatom enthaltenen Ring bilden können - substituierten Carbamoyl-oder einen $C_1$-$C_6$-Acylamino- oder $C_1$-$C_6$-Alkylaminorest substituierten 5- oder 6-Ring, der gegebenenfalls ein weiteres Stickstoff-, Sauerstoff-, Schwefelatom oder eine Carbonylgruppe enthält,

X ein Wasserstoffatom und/oder ein Metallionenäquivalent,

Y eine

COOX- oder 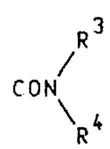 -Gruppe

bedeuten, sowie deren Salze mit organischen und/oder anorganischen Basen.

Verbindungen, die aus dem Anion eines dieser komplexbildenden Amide und einem oder mehreren Zentralionen eines Elements der Ordnungszahlen 21-29, 31, 32, 38, 39, 42-44, 49, 57-83 und gegebenenfalls einem oder mehreren Kationen einer anorganischen und/oder organischen Base oder Aminosäure bestehen, eignen sich zur Herstellung von NMR-, Röntgen- und Radio-Diagnostika.

Es wurde nun gefunden, daß überraschenderweise Verbindungen dieser allgemeinen Formel I, wenn sie mindestens ein Element der Ordungszahlen 21-29, 42, 44 od 58-70 enthalten, d.h. mindestens zwei der Substituenten X müssen für ein Metallionenäquivalent dieser Elemente stehen, insbesondere jedoch Verbindung der allgemeinen Formel I, worin

n die Ziffer 1,

$R^1$, $R^2$ Wasserstoffatome

$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, wenigstens eine $R^3$-Gruppe, eine Cycloalkyl-oder eine gegebenenfalls durch eine oder mehrere Di-$C_1$-$C_6$-alkylamino- oder durch eine oder

mehrere $C_1$-$C_6$-Alkoxygruppen substituierte Aryl- oder Aralkylgruppe,
$R^4$ ein Wasserstoffatom, ein gesättigter, ungesättigter, gerad- oder verzweigtkettiger oder cyclischer Kohlenwasserstoffrest mit bis zu 16 C-Atomen,
X ein Wasserstoffatom und/oder ein Metallionenäquivalent mindestens eines Elements der Ordnungszahlen 21-29, 42, 44 oder 58-70,
Y eine

COOX- oder 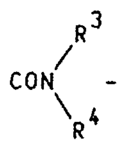 -Gruppe

bedeuten mit der Maßgabe, daß mindestens zwei der Substituenten X für ein Metallionenäquivalent stehen, sowie deren Salze mit organischen und/oder anorganischen Basen, ein unerwartetes pharmakokinetisches Verhalten zeigen.

So verteilt sich z.B. Magnevist(R) das weltweit bisher einzige zugelassene NMR-Kontrastmittel, nach intravenöser Injektion extrazellulär und wird durch glomeruläre Sekretion über die Nieren ausgeschieden. Eine Passage intakter Zellmembranen und eine extrarenale Ausscheidung werden praktisch nicht beobachtet.

Magnevist(R) ist besonders gut für die Diagnose pathologischer Bereiche (z.B. Entzündungen, Tumore, Infarkte etc.) geeignet.

Vor allem für Patienten mit eingeschränkter (Nierenfunktion (Niereninsuffizienz), bei denen Magnevist(R) nur sehr langsam ausgeschieden wird und zum Teil nur mit Hilfe eines Dialysegerätes aus dem Organismus entfernt werden kann, wären Kontrastmittel, die eine zumindest teilweise extrarenale Ausscheidung aufweisen, wünschenswert.

Es besteht daher ein Bedarf an NMR-Kontrastmitteln, die ein anderes pharmakokinetisches Verhalten und damit eine höhere Organspezifität als Magnevist(R) zeigen.

Der Erfindung liegt somit die Aufgabe zugrunde, derartige Verbindungen und Mittel zur Verfügung zu stellen. Diese Aufgabe wird durch die vorliegende Erfindung gelöst.

Es wurde gefunden, daß die o.g. Verbindungen überraschenderweise die gewünschte Eigenschaft zeigen: sowohl renale Ausscheidung als auch Exkretion mit den Faeces.

Überraschenderweise ist die Elimination über die Galle jedoch nicht der einzige extrarenale Ausscheidungsweg: bei NMR-Untersuchungen an Ratten wurde nach intravenöser Gabe der erfindungsgemäßen Verbindungen unerwartet auch eine Kontrastverstärkung des Magen-Darm-Traktes beobachtet. Die Nieren sowie implantierte Tumore werden ebenfalls besser kontrastiert.

Die Ausscheidung (Sekretion) über den Magen hat den Vorteil, daß eine Abgrenzung abdominaler Strukturen (z.B. Pankreas) vom Gastrointestinal-Trakt mit gleichzeitiger Kontrastverstärkung pathologischer Prozesse (Tumoren, Entzündungen) ermöglicht wird. Eine Darstellung des renalen Systems, der Leber und der Gallenblase und Gallenwege kann darüber hinaus auch erzielt werden. Neben der verbesserten Darstellung von Ulci und Magenkarzinomen kann auch eine Überprüfung der Magensaftsekretion mit Hilfe bildgebender Verfahren durchgeführt werden.

Durch die Zurverfügungstellung der erfindungsgemäßen Verbindungen kann somit sowohl niereninsuffizienten als auch den unter gastrointestinalen Erkrankungen leidenden Patienten (mindestens 10 % der Bevölkerung in den westlichen Industrieländern) geholfen werden. Die meisten dieser Patienten sowie eine große Anzahl von Patienten, bei denen der Verdacht auf eine solche Erkrankung vorliegt, müssen sich diagnostischen Untersuchungen unterziehen. Zur Zeit sind vor allem zwei dafür geeignete Methoden gebräuchlich: Die Endoskopie und die Röntgenstrahl-Diagnostik mit Hilfe von Barium-Kontrastmitteln.

Diese Untersuchungen weisen verschiedene Nachteile auf: sie sind mit dem Risiko der Strahlenbelastung behaftet, trauma-verursachend, mit Unannehmlichkeiten, gelegentlich sogar mit Risiken für den Patienten verbunden und können daher psychologischen Streß hervorrufen. Sie müssen meist wiederholt durchgeführt werden, sind relativ aufwendig durchzuführen, erfordern die aktive Mitarbeit des Patienten (z.B. Einnehmen einer bestimmten Körperhaltung) und sind oft nicht anwendbar bei gebrechlichen und bei Risiko-Patienten.

Die Aufgabe, neue diagnostische Methoden zur Erkennung und Lokalisierung gastrointestinaler Erkrankungen, die diese Nachteile nicht besitzen, zur Verfügung zu stellen, wird daher durch die Verwendung der o.g. Komplexverbindungen und Mittel ebenfalls gelöst.

Auch ohne spezifische Maßnahmen erlaubt ihre Pharmakokinetik die Verbesserung der Diagnose

zahlreicher Erkrankungen. Die Komplexe werden zum größten Teil unverändert und rasch wieder ausgeschieden, so daß insbesondere auch im Falle der Verwendung relativ toxischer Metallionen auch bei hoher Dosierung keine schädlichen Wirkungen beobachtet werden.

Die praktische Anwendung der neuen Komplexe wird auch durch deren günstige chemische Stabilität erleichtert.

Für die Anwendung in der NMR-Diagnostik muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium-(III)-, Holmium(III)-, Erbium(III)- und Eisen(III)-ion.

Als Alkylsubstituenten $R^1$ und $R^2$ kommen Kohlenwasserstoffe mit 1 - 8, vorzugsweise 1 - 4 C-Atomen, wie z.B. Methyl-, Ethyl-, Propyl-, Isopropyl, n-, sek.- oder tert.-Butyl-, Isobutyl-, Pentyl- und Hexyl-Reste, in Betracht.

Als Alkylsubstituenten $R^3$ und $R^4$ kommen gesättigte, ungesättigte, gerad-oder verzweigtkettige oder cyclische Kohlenwasserstoffe mit bis zu 16 C-Atomen, vorzugsweise gesättigte Kohlenwasserstoffe mit 1 bis 10 C-Atomen, insbesondere gesättigte Kohlenwasserstoffe mit 1 bis 5 C-Atomen in Betracht. Beispielsweise seien genannt: Methyl, Ethyl, Propyl, Isopropyl, Butyl, Isobutyl, Pentyl, Cyclopentyl, Cyclohexyl, Propenyl.

Falls $R^4$ für ein Wasserstoffatom steht, kann $R^3$ auch eine gegebenenfalls durch eine oder mehrere (bis zu drei) Di-$C_1$- bis $C_6$-alkylamino- oder durch eine oder mehrere (bis zu drei) $C_1$- bis $C_6$-Alkoxygruppen substituierte $C_6$-$C_{10}$-Aryl- oder $C_1$-$C_{10}$-Ar-$C_1$-$C_6$-alkylgruppe, beispielsweise Phenyl- oder Benzylgruppe bedeuten.

Der durch $R^3$ und $R^4$ unter Einschluß des Amid-Stickstoffs gebildete heterocyclische 5- oder 6-Ring kann gesättigt, ungesättigt und/oder substituiert sein und gegebenenfalls ein Stickstoff-, Sauerstoff-, Schwefelatom oder eine Carbonylgruppe enthalten.

Der Heterocyclus kann substituiert sein durch eine Hydroxy-, eine $C_1$-$C_6$-Alkylgruppe, beispielsweise Methyl, Ethyl, Propyl, Isopropyl, Butyl, eine $C_1$-$C_5$-Hydroxyalkylgruppe, beispielsweise Hydroxymethyl, Hydroxyethyl, oder durch eine $C_2$-$C_6$-Acylgruppe, zum Beispiel Acetyl, Propionyl, die gegebenenfalls durch eine Hydroxy- oder $C_1$-$C_6$-Alkoxygruppe, beispielsweise Methoxy, Ethoxy, substituiert sein kann.

Als weiterer Substituent sei die Carbamoylgruppe genannt, die direkt oder durch eine $C_1$-$C_6$-Alkylengruppe, beispielsweise Methylen, Ethylen, Propylen getrennt am Heterocyclus gebunden ist und gegebenenfalls durch einen oder zwei $C_1$-$C_6$-Alkylrest(e), beispielsweise Methyl, Ethyl, Propyl, Isopropyl, die gegebenenfalls einen Ring wie zum Beispiel einen Pyrrolidin- oder Piperidin-Ring bilden, am Stickstoff substituiert ist. Der Carbamoyl-Stickstoff kann auch Bestandteil eines Morpholin-Ringes sein.

Als weiterer möglicher Substituent am Heterocyclus sei eine gegebenenfalls $C_1$-$C_6$-alkylierte oder $C_1$-$C_6$-acylierte primäre oder sekundäre Aminogruppe, wie beispielsweise die Methyl-, Ethyl-, Acetyl-, Propionyl-Aminogruppe, genannt.

Wenn der Heterocyclus substituiert ist, beträgt die Gesamtzahl der Substituenten 1 bis 3.

Als geeignete Heterocyclen seien beispielhaft genannt: der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Wenn nicht alle aciden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Die Einführung von Amidgruppen zur Herstellung der Komplexbildner, d.h. von Verbindungen der allgemeinen Formel I mit X in der Bedeutung von Wasserstoff, erfolgt durch partielle Umwandlung von aktivierten Carboxylgruppen in Amidgruppen der - entsprechend dem gewünschten Endprodukt -jeweils geeigneten Tetra-, Penta- bzw. Hexacarbonsäuren. Für diesen Prozeß kommen alle dem Fachmann bekannten Synthesemöglichkeiten in Betracht.

Ein Beispiel hierfür ist die Umsetzung der Anhydride oder Ester der allgemeinen Formeln II, IV, V und VI:

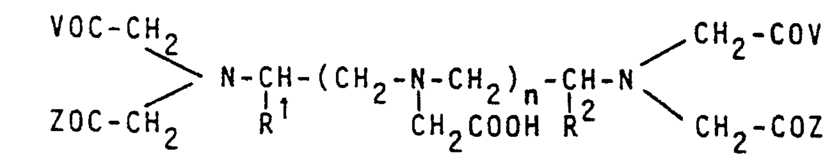

(II)

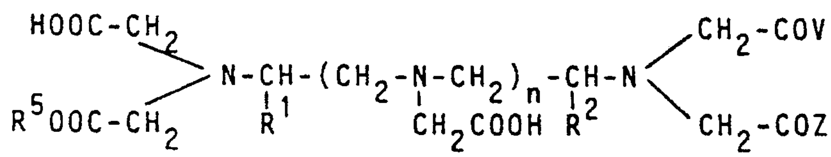

(IV)

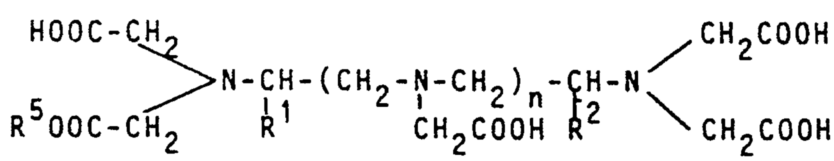

(V)

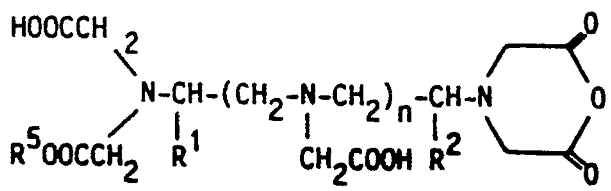

(VI),

worin
$R^1$, $R^2$ und n die oben angegebenen Bedeutungen haben, V und Z gemeinsam ein Sauerstoffatom oder V eine Hydroxygruppe und Z die Gruppierung $OR^5$ und $R^5$ einen $C_1$-$C_6$-Alkylrest darstellen, mit Aminen der allgemeinen Formel III

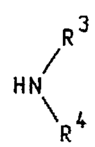

(III),

worin
$R^3$ und $R^4$ die oben genannten Bedeutungen haben.

Als geeignete Amine seien beispielsweise genannt: Dimethylamin, Diethylamin, Di-n-propylamin, Diisopropylamin, Di-n-butylamin, Diisobutylamin, Di-sek.-butylamin, N-Methyl-n-propylamin, Dioctylamin, Dicyclohexylamin, N-Ethylcyclohexylamin, Diisopropenylamin, Benzylamin, Anilin, 4-Methoxyanilin, 4-Dimethylaminoanilin, 3,5-Dimethoxyanilin, 4-Methoxybenzylamin, Morpholin, Pyrrolidin, Piperidin, N-Methyl-piperazin, N-Ethylpiperazin, N-(2-Hydroxyethyl)-piperazin, N-(Hydroxymethyl)-piperazin, Piperazinoessigsäureisopropylamid, N-(Piperazinomethylcarbonyl)morpholin, N-(Piperazinomethylcarbonyl)-pyrrolidin, 2-(2-Hydroxymethyl)-piperidin, 4-(2-Hydroxyethyl)-piperidin, 2-Hydroxymethylpiperidin, 4-Hydroxymethylpiperidin, 2-Hydroxymethyl-pyrrolidin, 3-Hydroxy-piperidin, 4-Hydroxy-piperidin, 3-Hydroxy-pyrrolidin, 4-Piperidon, 3-Pyrrolin, Piperidin-3-carbonsäureamid, Piperidin-4-carbonsäureamid, Piperidin-3-carbonsäurediethylamid, Piperidin-4-carbonsäuredimethylamid, 2,6-Dimethylpiperidin, 2,6-Dimethylmorpholin, N-Acetyl-piperazin, N-(2-Hydroxy-propionyl)-piperazin, N-(3-Hydroxy-propionyl)-piperazin, N-(Methoxyacetyl)-piperazin, 4-(N-Acetyl,N-methylamino)-piperidin, Piperidin-4-carbonsäure-(3-oxapentamethylen)-amid, Piperidin-3-carbonsäure-(3-oxapentamethylen)-amid, N-(N′,N′-Dimethyl-carbamoyl)-piperazin, Pyrazolin, Pyrazolidin, Imidazolin, Oxazolidin, Thiazolidin.

Die Verseifung gegebenenfalls noch vorhandener Estergruppen erfolgt nach den dem Fachmann bekannten Verfahren, beispielsweise durch alkalische Hydrolyse.

Die Herstellung der Säureanhydride der allgemeinen Formel II kann nach bekannten Verfahren erfolgen, z.B. nach der im US-Patent 3.660.388 bzw. in DE-OS 1 695 050 beschriebenen Verfahrensweise mit Acetanhydrid in Pyridin. In bestimmten Fällen ist es jedoch von besonderem Vorteil, die Wasserabspaltung mit Carbodiimiden in einem geeigneten Lösungsmittel, wie z.B. Dimethylformamid oder Dimethylacetamid, schonend vorzunehmen.

Die Herstellung der Monoanhydride der allgemeinen Formel VI soll am Beispiel des Monoanhydrids des Diethylentriaminpentaessigsäure-ethylesters, ausgehend vom Monoethylester der DTPA (J. Pharm. Sci. 68 , 1979, 194), beschrieben werden:

$N^3$-(2,6-Dioxomorpholinoethyl)-$N^6$-(ethoxycarbonylmethyl)-3,6-diazaoctandisäure

Eine Suspension von 21,1 g (50 mMol) $N^3$,$N^6$-Bis-(carboxymethyl)-$N^9$-(ethoxycarbonylmethyl)-3,6,9-triazaundecandisäure in 250 ml Essigsäureanhydrid läßt man nach Zugabe von 42,2 ml Pyridin 3 Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40 °C erhält man 18,0 g (= 89 % der Theorie) eines weißen Pulvers vom Schmelzpunkt 195 - 196 °C.

| Analyse (bezogen auf wasserfreie Substanz): | | | |
|---|---|---|---|
| Berechnet: | C 47,64 | H 6,25 | N 10,42 |
| Gefunden: | C 47,54 | H 6,30 | N 10,22 |

Die Umsetzung der Säureanhydride zu den Amiden wird in flüssiger Phase durchgeführt. Geeignete Reaktionsmedien sind beispielsweise Wasser, dipolare aprotische Lösungsmittel wie Acetonitril, N-Methylpyrrolidon, Dimethylformamid, Dimethylacetamid und dergleichen oder Gemische derselben. Die Reaktionstemperaturen liegen zwischen ca. 0 °C - 100 °C, wobei Temperaturen von 20 °C - 80 °C bevorzugt sind. Die Reaktionszeiten liegen zwischen 0,5 Stunden und 2 Tagen, vorzugsweise zwischen 1 Stunde und 36 Stunden.

Die Herstellung der Ester der allgemeinen Formel V erfolgt in bekannter Weise, z.B. nach den in R.A. Guilmette et al., J. Pharm. Sci. 68 , 194 (1979) beschriebenen Verfahren.

Die Aminolyse der Ester erfolgt in flüssiger Phase, z.B. in einem geeigneten höhersiedenden Lösungsmittel wie Dimethylformamid, Dimethylacetamid oder Dimethylsulfoxid. Die Reaktionstemperaturen liegen bei etwa 20 °C - 200 °C, wobei Temperaturen von 100 °C - 180 °C bevorzugt sind. Die Reaktionszeiten liegen zwischen 2 Stunden und 2 Tagen, wobei Reaktionszeiten zwischen 4 Stunden und 36 Stunden bevorzugt sind.

Darüber hinaus können alle dem Fachmann bekannten Methoden zur Umwandlung von Carboxylgruppen in Amidgruppen zur Synthese der erfindungsgemäßen Komplexbildner der allgemeinen Formel I herangezogen werden, so z.B. die Methode nach Krejcarek und Tucker, Biochem. Biophys. Res. Commun. 77 , 581 (1977) über gemischte Anhydride.

Die so erhaltenen Verbindungen der allgemeinen Formel I mit X in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten X in der Bedeutung eines Metallionenäquivalents überführt werden.

Die Herstellung der Metallkomplexe erfolgt in der Weise, wie sie in den Patentschriften EP 71564, EP 130934 und DE-OS 3 401 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 42, 44 oder 58-70 in Wasser und/oder einem niederen Alkohol (wie Methanol, Ethanol oder Isopropanol) löst oder suspendiert und mit der Lösung oder Suspension der äquivalenten Menge der komplexbildenden Säure der allgemeinen Formel I mit X in der Bedeutung eines Wasserstoffatoms umsetzt und anschließend, falls gewünscht, vorhandene acide Wasserstoffatome von Säuregruppen durch Kationen anorganischer und/oder organischer Basen oder Aminosäuren substituiert.

Die Neutralisation erfolgt dabei mit Hilfe anorganischer Basen (z.B. Hydroxiden, Carbonaten oder Bicarbonaten) von z.B. Natrium, Kalium oder Lithium und/oder organischer Basen wie unter anderem

primärer, sekundärer und tertiärer Amine, wie z.B. Ethanolamin, Morpholin, Glucamin, N-Methyl-und N,N-Dimethylglucamin, sowie basischer Aminosäuren, wie z.B. Lysin, Arginin und Ornithin.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol etc.), niederen Ketonen (Aceton etc.), polaren Ethern (Tetrahydrofuran, Dioxan, 1,2-Dimethoxyethan etc.) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls aufreinigt und dann zur vollständigen Neutralisation mit der benötigten Menge anorganischer Base versetzt. Die Reihenfolge der Basenzugabe kann auch umgekehrt werden.

Die Herstellung der diagnostischen Mittel erfolgt ebenfalls in an sich bekannter Weise, indem man die erfindungsgemäßen Komplexverbindungen -gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie z.B. Tromethamin), geringe Zusätze von Komplexbildnern (wie z.B. Diethylentriaminpentaessigsäure) oder, falls erforderlich, Elektrolyte wie z.B. Natriumchlorid oder, falls erforderlich, Antioxidantien wie z.B. Ascorbinsäure.

Sind für die enterale Verabreichung oder andere Zwsecke Suspensionen oder Lösungen der erfindungsgemäßen Mittel in Wasser oder physiologischer Salzlösung erwünscht, werden sie mit einem oder mehreren in der Galenik üblichen Hilfsstoffen (z.B. Methylcellulose, Lactose, Mannit) und/oder Tensiden (z.B. Lecithine, Tweens$^{(R)}$, Myrj$^{(R)}$ und/oder Aromastoffen zur Geschmackskorrektur (z.B. ätherischen Ölen) gemischt.

Prinzipiell ist es auch möglich, die erfindungsgemäßen diagnostischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Für die erfindungsgemäße Diagnostik in der Kernspintomographie werden die diagnostischen Mittel in einer Dosis von 1 µmol/kg bis 5 mmol/kg, vorzugsweise von 10 µmol bis 0,5 mmol/kg des erfindungsgemäßen Komplexsalzes appliziert. Bei einer intravenösen Injektion finden wäßrige Formulierungen der Konzentration 50 µmol/l bis 2 mol/l, vorzugsweise 100 mmol/l bis 1 mol/l, Verwendung. Eine rektale sowie orale Anwendung wird vorzugsweise mit Lösungen der Konzentration 0,1 mmol bis 100 mmol/l durchgeführt. Die applizierten Volumina sind je nach diagnostischer Fragestellung zwischen 5 ml und 2 l.

Die diagnostischen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der diagnostischen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, d.h. NMR-Diagnostika müssen 100 - 1000fach besser wasserlöslich sein als für die in-vitro-NMR-Spektroskopie. Weiterhin weisen die erfindungsgemäßen Mittel nicht nur eine hohe Stabilität in vitro auf, sondern auch eine überraschend hohe Stabilität in vivo, so daß eine Freigabe oder ein Austausch der in den Komplexen nicht kovalent gebundenen - an sich giftigen - Ionen innerhalb der Zeit, in der die Kontrastmittel vollständig wieder ausgeschieden werden, nur äußerst langsam

erfolgt.

Die erfindungsgemäßen Mittel können auch für die Strahlentherapie verwendet werden. So sind Komplexe des Gadoliniums aufgrund des großen Einfangquerschnitts für die Neutroneneinfangtherapie hervorragend geeignet. Ist das erfindungsgemäße Mittel zur Anwendung in der von R.L. Mills et al. [Nature Vol. 336 (1988) S. 787] vorgeschlagenen Variante der Strahlentherapie bestimmt, so muß sich das Zentralion von einem Mößbauer-Isotop wie z.B. $^{57}Fe$ oder $^{151}Eu$ ableiten.

Bei der Applikation der erfindungsgemäßen Mittel können diese auch zusammen mit einem geeigneten Träger wie z.B. Serum oder physiologischer Kochsalzlösung und/oder zusammen mit einem Protein wie z.B. Human Serum Albumin verabreicht werden. Die Dosierung ist dabei abhängig von der Art der zellulären Störung und den Eigenschaften des zur Anwendung kommenden Metallkomplexes.

Insgesamt ist es somit gelungen, mit den genannten Komplexverbindungen neue Möglichkeiten in der diagnostischen Medizin zu erschließen.

Die nachfolgenden Beispiele dienen zur Erläuterung des Erfindungsgegenstandes, ohne ihn auf diese beschränken zu wollen.

**Beispiel 1**

a) 6-Carboxymethyl-3-ethoxycarbonylmethyl-9-phenylaminocarbonylmethyl-3,6,9-triazaundecandisäure

2,42 g (6 mmol) $N^3$-(2,6-Dioxomorpholinoethyl)-$N^6$-(ethoxycarbonylmethyl)-3,6-diazaoctandisäure werden in DMF bei 0 °C mit 4,16 ml (3,04 g, 30 mmol) Triethylamin und 559 mg (6 mmol) Anilin versetzt und über Nacht bei Raumtemperatur gerührt. Anschließend wird die klare Lösung im Vakuum eingeengt und der Rückstand an Kieselgel mit Dichlormethan/Methanol/Essigsäure/Wasser (5:3:1:1) als Laufmittel chromatographiert. Die vereinigten Fraktionen werden über ca. 10 ml Amberlite IR 120 ($H^+$-Form) gegeben und das saure Eluat eingeengt.
Ausbeute 2,35 g (79 %)

| | | | |
|---|---|---|---|
| Berechnet: | C 53,21 | H 6,50 | N 11,29 |
| Gefunden: | C 53,01 | H 6,55 | N 11,26 |

b) 3,6,9-Tris(carboxymethyl)-3,6,9-triazaundecandisäure-phenylmonoamid

1,5 g (3mmol) des in Beispiel a) beschriebenen Ethylesters werden in 2 n NaOH gelöst und 2 Stunden bei Raumtemperatur gerührt. Durch Zugabe von Amberlite IR 120 ($H^+$-Form) wird auf pH 7 gestellt, abfiltriert und die neutrale Lösung über ca. 16 ml Amberlite IR 120 ($H^+$) gegeben. Das saure Eluat wird eingeengt und bei 50 °C im Vakuum nachgetrocknet.
Ausbeute: 1,3 g (92,5 %)

| | | | |
|---|---|---|---|
| Berechnet: | C 51,27 | H 6,03 | N 11,96 |
| Gefunden: | C 51,19 | H 5,99 | N 11,91 |

c) Gadoliniumkomplex des 3,6,9-Tris(carboxymethyl)-3,6,9-triazaundecandisäure-phenylmonoamids

936 mg (2 mmol) der nach Beispiel 1b) erhaltenen Komplexbildnersäure werden in ca. 40 ml Wasser gelöst und bei 80 °C mit 362 mg (1 mmol) $Gd_2O_3$ versetzt. Nach 30 Minuten wird die fast klare Lösung filtriert und das Filtrat gefriergetrocknet.
Ausbeute: 1,23 g (98,8 %), bezogen auf die wasserfreie Substanz.

| | | | | |
|---|---|---|---|---|
| Berechnet: | C 38,57 | H 4,05 | N 9,00 | Gd 25,25 |
| Gefunden: | C 38,33 | H 4,10 | N 9,03 | Gd 24,99 |

**Beispiel 2**

Herstellung einer Lösung des N-Methylglucaminsalzes des Gadoliniumkomplexes von 3,6,9-Tris-(carboxymethyl)-3,6,9-triazaundecandisäure-phenylmonoamid

1,87 g (3 mmol) des Gadoliniumkomplexes von 3,6,9-Tris(carboxymethyl)-3,6,9-triazaundecandisäure-phenylmonoamid (Beispiel 1) werden in 5 ml Wasser pro injectione suspendiert und mit 0,586 g (3 mmol) N-Methylglucamin versetzt, wobei der Komplex in Lösung geht. Man füllt mit Wasser auf 10 ml auf, gibt die Lösung in ein Vial und unterzieht sie der Hitzesterilisation.

Die $T_1$-Relaxivität (1/mmol sec) beträgt:

in Wasser: 3,79 ± 0,16

in Plasma: 5,45 ± 0,68

**Beispiel 3**

Herstellung einer Lösung des Natriumsalzes des Gadolinium-III-Komplexes des 3,6,9-Tris(carboxymethyl)-3,6,9-triazaundecandisäure-phenylmonoamids

62,27 g (0,1 mol) des nach Beispiel 1c) erhaltenen Gadoliniumkomplexes werden in 800 ml Wasser pro injectione (p.i.) suspendiert und durch tropfenweise Zugabe von n-Natronlauge bei pH 7,2 in Lösung gebracht. Nach Zugabe von 0,2 g Tromethamin wird mit Wasser p.i. auf 1000 ml aufgefüllt, die Lösung in Flaschen abgefüllt und hitzesterilisiert.

**Beispiel 4**

a) 3,6,9-Tris-(carboxymethyl)-3,6,9-triazaundecandisäure-(N,N′-diphenyl)-diamid

Man suspendiert 42,88 g (120 mmol) DTPA-bis-anhydrid in 330 ml Dimethylformamid und kühlt im Eisbad unter Rühren auf ca. 5 °C. Innerhalb von 50 Minuten wird eine Lösung von 32,9 ml (360 mmol) Anilin in 30 ml Dimethylformamid zugetropft. Man rührt noch 1 Stunde im Eisbad, dann über Nacht bei Raumtemperatur. Nach dieser Zeit hat sich eine leicht trübe Lösung gebildet. Das Lösungsmittel wird im Vakuum entfernt und der schmierige Rückstand zur Entfernung von Lösungsmittelresten mit Diethylether verrieben. Der Rückstand wird mit 500 ml Wasser versetzt und durch Zugabe von 20 ml 11n Natronlauge gelöst. Die Lösung wird mit 3,5 g Aktivkohle versetzt, filtriert und gefriergetrocknet. Man erhält 73,9 g des Natriumsalzes der Titelverbindung als Pulver.

b) Gadoliniumkomplex des 3,6,9-Tris-(carboxymethyl)-3,6,9-triazaundecandisäure-(N,N′-diphenyl)-diamids

10,9 g Gadoliniumoxid (30 mmol) werden mit 10,6 ml Eisessig und 150 ml Wasser 20 Minuten unter Rückfluß erhitzt. Man filtriert die Lösung über ein 0,1 µ Membranfilter, versetzt mit 35,3 g (60 mmol) des nach a) erhaltenen Liganden und erhitzt 90 Minuten auf 80 °C. Die Lösung wird mit 2,1 g Aktivkohle 30 Minuten gerührt, filtriert und dann nacheinander über eine Anionenaustauschersäule (200 ml IRA-410) und 100 ml Kationenaustauscher (IRC-50) gegeben. Die Eluate von den Säulen werden über ein 0,1 µ Membranfilter filtriert und gefriergetrocknet. Man erhält 17,6 g der Titelverbindung als weißes Pulver.

| Analyse: | | | | |
|---|---|---|---|---|
| Berechnet: | C 44,75 | H 4,33 | Gd 22,54 | N 10,04 |
| Gefunden: | C 44,60 | H 4,44 | Gd 22,42 | N 9,89 |

**Beispiel 5**

a) 3,6,9-Tris-(carboxymethyl)-3,6,9-triazaundecandisäure-(N,N′-dibenzyl)-diamid

Man suspendiert 42,88 g (120 mmol) DTPA-bis-anhydrid in 330 ml Dimethylformamid und kühlt im Eisbad unter Rühren auf 5 °C. Innerhalb von 1 Stunde tropft man eine Lösung von 39,3 ml (360 mmol) Benzylamin in 30 ml Dimethylformamid zu. Man rührt noch 1 Stunde im Eisbad, dann über Nacht bei Raumtemperatur. Nach Entfernen des Lösungsmittels im Vakuum wird der Rückstand mit Diethylether verrieben, mit 500 ml Wasser versetzt und durch Zugabe von 20 ml 11 n Natronlauge in Lösung gebracht. Nach dem Gefriertrocknen erhält man 71 g des Natriumsalzes der Titelverbindung als hellgelbes Pulver.

b) Gadoliniumkomplex des 3,6,9-Tris-(carboxymethyl)-3,6,9-triazaundecandisäure-(N,N′-dibenzyl)-diamids

10,9 g Gadoliniumoxid (30 mmol) werden mit 10,6 ml Eisessig und 150 ml Wasser 20 Minuten unter Rückfluß erhitzt. Man filtriert die Lösung über ein 0.1 µ Membranfilter, versetzt mit 37 g (60 mmol) des nach 5a) erhaltenen Liganden und erhitzt 90 Minuten auf 80 °C. Die Lösung wird mit 3 g Aktivkohle 30 Minuten gerührt, filtriert und dann nacheinander über eine Anionen-Austauschersäule (200 ml IRA-410) und 100 ml Kationen-Austauschersäule (100 ml IRC-50) gegeben. Die Eluate werden über ein 0.1 µ Membranfilter filtriert und gefriergetrocknet. Man erhält 18 g der Titelverbindung als weißes Pulver.

| Analyse: | | | | |
|---|---|---|---|---|
| Berechnet: | C 46,33 | H 4,72 | Gd 21,66 | N 9,65 |
| Gefunden: | C 46,46 | H 4,49 | Gd 21,50 | N 9,81 |

**Beispiel 6**

a) 6-Carboxymethyl-3-ethoxycarbonylmethyl-9-benzylaminocarbonylmethyl-3,6,9-triazaundecandisäure

Man suspendiert 5,04 g (12,5 mmol) $N^3$-(2,6-Dioxomorpholinoethyl)-$N^6$-(ethoxycarbonylmethyl)-3,6-diazaoctandisäure in 65 ml Dimethylformamid, rührt im Eisbad und versetzt nacheinander mit 8,7 ml (62,5 mmol) Triethylamin und 1,34 g (12,5 mmol) Benzylamin. Man rührt dann noch 2 Stunden im Eisbad, anschließend über Nacht bei Raumtemperatur. Das Lösungsmittel wird im Vakuum abdestilliert, der Rückstand mit Diisopropylether gerührt, abgesaugt und getrocknet. Zur weiteren Reinigung löst man den Rückstand in soviel 11 n Natronlauge, daß gerade ein pH-Wert von 7 erreicht wird. Dazu gibt man 5 g Kieselgel, trocknet die Suspension im Vakuum und gibt den Rückstand auf eine Säule von 350 g Kieselgel, die mit einer Mischung aus Chloroform / Methanol / Eisessig / Wasser (1750/1050/350/350) aufgezogen wurde. Man eluiert mit dem gleichen Lösungsmittel und erhält nach Abdampfen des Lösungsmittels 4,98 g einer farblosen Schmiere, die man in 35 ml Wasser löst und über eine Säule von 35 ml des Kationen-Austauschers IR 120 laufen läßt. Die Säule wird mit 70 ml Wasser gewaschen, die vereinigten Eluate im Vakuum eingedampft und der Rückstand mit Diethylether verrieben. Man erhält 2,65 g der Titelverbindung als weißes Pulver.

| Analyse: | | | |
|---|---|---|---|
| Berechnet: | C 54,11 | H 6,71 | N 10,97 |
| Gefunden: | C 53,95 | H 6,88 | N 11,23 |

b) 3,6,9-Tris(carboxymethyl)-3,6,9-triazaundecandisäure-benzylmonoamid

Eine Lösung von 2,26 g der nach Beispiel 6a) hergestellten Substanz in 46 ml 1n Natronlauge läßt man 2,5 Stunden bei Raumtemperatur stehen und gibt die Lösung dann über eine Säule aus 110 ml Kationenaustauscher IR 120. Man eluiert zunächst mit 200 ml Wasser und verwirft diese Fraktion. Man eluiert nun mit 600 ml 0,5n Ammoniak, stellt dieses Eluat durch Zugabe von IR 120 auf einen pH-Wert von 2,3 ein und unterwirft die Lösung einer Gefriertrocknung. Man erhält 1,50 g der Titelverbindung als weißes Pulver.

| Analyse: | | | |
|---|---|---|---|
| Berechnet: | C 52,28 | H 6,27 | N 11,61 |
| Gefunden: | C 52,44 | H 6,32 | N 11,80 |

c) Gadoliniumkomplex des 3,6,9-Tris(carboxymethyl)-3,6,9-triazaundecandisäure-benzylmonoamids

965 mg (2 mmol) der nach Beispiel 6b) hergestellten Substanz werden mit 40 ml Wasser und 362 mg (1 mmol) Gadoliniumoxid 1 Stunde auf 80 °C erhitzt. Man kühlt auf Raumtemperatur, filtriert die Lösung durch ein 0,1 μ Membranfilter und isoliert die Substanz durch Gefriertrocknen. Man erhält 1,15 g der Titelverbindung als weißes Pulver.

| Analyse: | | | | |
|---|---|---|---|---|
| Berechnet: | C 39,61 | H 4,27 | Gd 24,70 | N 8,80 |
| Gefunden: | C 39,50 | H 4,48 | Gd 24,61 | N 8,97 |

**Beispiel 7**

Dysprosium-(III)-Komplex von 3,6,9-Tris(carboxymethyl)-3,6,9-triazaundecandisäure-benzylmonoamid

965 mg (2 mmol) 3,6,9-Tris(carboxymethyl)-3,6,9-triazaundecandisäure-benzylmonoamid (Beispiel 6b) werden mit 40 ml Wasser und 373 mg (1 mmol) Dysprosium-(III)-oxid 1 Stunde auf 80 °C erhitzt. Man kühlt auf Raumtemperatur ab, filtriert die Lösung durch ein 0,1 μ Membranfilter und isoliert die Substanz durch Gefriertrocknen. Man erhält 1,22 g der Titelverbindung als weißes Pulver.

| Analyse: | | | | |
|---|---|---|---|---|
| Berechnet: | C 39,29 | H 4,24 | Dy 25,31 | N 8,73 |
| Gefunden: | C 39,41 | H 4,51 | Dy 25,19 | N 8,70 |

**Beispiel 8**

a) 6-Carboxymethyl-3-ethoxycarbonylmethyl-9-tert-butylaminocarbonylmethyl-3,6,9 triazaundecandisäure

2,42 g (6 mmol) $N^3$-(2,6-Dioxomorpholinoethyl)-$N^6$-(ethoxycarbonylmethyl)-3,6-diazaoctandisäure werden in DMF bei 0 °C mit 4,16 ml (3,04 g, 30 mmol) Triethylamin und 0,64 mg (6 mmol) tert.-Butylamin versetzt und über Nacht bei Raumtemperatur gerührt. Anschließend wird die klare Lösung im Vakuum eingeengt und der Rückstand an Kieselgel mit Dichlormethan/Methanol/Essigsäure/Wasser (5:3:1:1) als Laufmittel chromatographiert. Die vereinigten Fraktionen werden über ca. 10 ml Amberlite IR 120 ($H^+$-Form) gegeben und das saure Eluat eingeengt.
Ausbeute 2,14 g (75 %).

| | | | |
|---|---|---|---|
| Berechnet: | C 50,41 | H 7,62 | N 11,76 |
| Gefunden: | C 50,26 | H 7,66 | N 11,80 |

b) 3,6,9-Tris(carboxymethyl)-3,6,9-triazaundecandisäure-tert.-butylmonoamid

1,43 g (3 mmol) des in Beispiel a) beschriebenen Ethylesters werden in 2 n NaOH gelöst und 2 Stunden bei Raumtemperatur gerührt. Durch Zugabe von Amberlite 120 ($H^+$-Form) wird auf pH 7 gestellt, abfiltriert und die neutrale Lösung über ca. 16 ml Amberlite IR 120 ($H^+$) gegeben. Das saure Eluat wird eingeengt und bei 50 °C im Vakuum nachgetrocknet.
Ausbeute: 1,20 g (89 %)

| | | | |
|---|---|---|---|
| Berechnet: | C 48,21 | H 7,19 | N 12,49 |
| Gefunden: | C 48,13 | H 7,24 | N 12,41 |

c) Gadoliniumkomplex des 3,6,9-Tris(carboxymethyl)-3,6,9-triazaundecandisäure-tert.-butylmonoamids

897 mg (2 mmol) der nach Beispiel 8b) erhaltenen Komplexbildnersäure werden in ca. 40 ml Wasser gelöst und bei 80 °C mit 362 mg (1 mmol) $Gd_2O_3$ versetzt. Nach 30 Minuten wird die fast klare Lösung filtriert und das Filtrat gefriergetrocknet.
Ausbeute: 1,19 g (99 %), bezogen auf die wasserfreie Substanz.

| | | | | |
|---|---|---|---|---|
| Berechnet: | C 35,87 | H 4,85 | N 9,30 | Gd 26,09 |
| Gefunden: | C 35,97 | H 4,79 | N 9,28 | Gd 25,83 |

**Beispiel 9**

a) 6-Carboxymethyl-3-ethoxycarbonylmethyl-9-(4-methoxybenzylcarbamoylmethyl)-3,6,9-triazaundecandisäure

14,10 g (35 mmol) $N^3$-(2,6-Dioxomorpholinoethyl)-$N^6$-(ethoxycarbonylmethyl)-3,6-diazaoctandisäure werden in 175 ml Dimethylformamid bei 0 °C mit 24,4 ml (175 mmol) Triethylamin und 4,67 ml (35 mmol) 4-Methoxybenzylamin versetzt und über Nacht bei Raumtemperatur gerührt. Das Lösungsmittel wird im

Vakuum weitgehend abdestilliert und der Rückstand mit 600 ml Diisopropylether zum Sieden erhitzt. Nach Abkühlen auf Raumtemperatur wird vom Lösungsmittel dekantiert. Der Rückstand wird in 185 ml Wasser gelöst und über eine Säule mit 140 ml Kationenaustauscher IR 120 ($H^+$-Form) gegeben und die Säule mit 200 ml Wasser gewaschen. Die vereinigten Eluate werden im Vakuum auf ein Drittel eingeengt und anschließend gefriergetrocknet. Man erhält 16,33 g der Titelverbindung als weißes Pulver, das noch 1,2 % Wasser und 1 % Dimethylformamid enthält.

| Analyse (nach Korrektur der Lösungsmittelanteile): | | | |
|---|---|---|---|
| Berechnet: | C 53,33 | H 6,71 | N 10,36 |
| Gefunden: | C 53,51 | H 6,78 | N 10,18 |

b) 3,6-Bis(carboxymethyl)-9-(4-methoxybenzyl-carbamoylmethyl)-3,6,9-triazaundecandisäure

12,34 g (22 mmol) des in Beispiel 9a) beschriebenen Ethylesters werden in 200 ml Wasser und 20 ml 11 N Natronlauge gelöst und 2 Stunden bei Raumtemperatur gerührt. Durch Zugabe von 140 ml Amberlite IR 120 ($H^+$-Form) erreicht man ph 2,3. Man filtriert und unterzieht die Lösung einer Gefriertrocknung.
Ausbeute: 9,61 g (85 % d.Th.), Wassergehalt 2,18 %.

| Analyse (nach Korrektur des Wassergehalts): | | | |
|---|---|---|---|
| Berechnet: | C 51,56 | H 6,29 | N 10,93 |
| Gefunden: | C 51,37 | H 6,44 | N 10,89 |

c) Gadoliniumkomplex der 3,6-Bis(carboxymethyl)-9-(4-methoxybenzylcarbamoylmethyl)-3,6,9-triazaundecandisäure

5,24 g (10 mmol) der nach Beispiel 9b) erhaltenen Komplexbildnersäure werden in 200 ml Wasser mit 1,81 g (5 mmol) Gadoliniumoxid 1 Stunde bei 80 °C gerührt, wobei eine fast klare Lösung entsteht. Man filtriert und unterzieht das Filtrat einer Gefriertrocknung.
Ausbeute: 6,31 g, Wassergehalt 3,7 %.

| Analyse (nach Korrektur des Wassergehalts): | | | | |
|---|---|---|---|---|
| Berechnet: | C 39,63 | H 4,38 | N 8,40 | Gd 23,99 |
| Gefunden: | C 39,88 | H 4,53 | N 8,51 | Gd 23,70 |

d) N-Methylglucaminsalz des Gadoliniumkomplexes der 3,6-Bis(carboxymethyl)-9-(4-methoxybenzylcarbamoylmethyl-3,6,9-triazaundecandisäure

2 g des nach Beispiel 9c) erhaltenen Gadoliniumkomplexes werden in 30 ml Wasser gelöst, mit 1 Equivalent N-Methylglucamin versetzt und die Lösung im Vakuum eingedampft.
Ausbeute: 2,30 g, Wassergehalt 4 %

| Analyse (nach Korrektur des Wassergehalts): | | | | |
|---|---|---|---|---|
| Berechnet:<br>Gefunden: | C 40,41<br>C 40,52 | H 5,38<br>H 5,11 | N 8,12<br>N 8,27 | Gd 18,24<br>Gd 18,39 |

e) Natriumsalz des Gadoliniumkomplexes der 3,6-Bis(carboxymethyl)-9-(4-methoxybenzyl-carbamoylmethyl)-3,6,9-triazaundecandisäure

Man löst 0,5 g des nach Beispiel 9c) erhaltenen Gadoliniumkomplexes in 10 ml Wasser, versetzt mit 1 Equivalent Natriumhydroxid gelöst in 5 ml Wasser und unterwirft die Lösung der Titelverbindung der Gefriertrocknung.
Man erhält 0,55 g der Titelverbindung als weißes Pulver mit einem Wassergehalt von 4,5 %.

| Analyse (nach Korrektur des Wassergehalts): | | | | | |
|---|---|---|---|---|---|
| Berechnet:<br>Gefunden: | C 38,37<br>C 38,40 | H 4,10<br>H 4,45 | Gd 22,83<br>Gd 22,43 | N 8,13<br>N 8,10 | Na 3,34<br>Na 3,57 |

**Beispiel 10**

a) 6-Carboxymethyl-3-ethoxycarbomylmethyl-9-(N-undecylcarbamoylmethyl)-3,6,9-triazaundecandisäure

12,10 g (30 mmol) $N^3$-(2,6-Dioxomorpholinoethyl)-$N^6$-(ethoxycarbonylmethyl)-3,6-diazaoctandisäure werden unter Stickstoffatmosphäre in 1 l absolutem Dimethylformamid bei 0 °C mit 12,6 ml (91 mmol) Triethylamin und 5,14 g (30 mmol) 1-Undecylamin versetzt und 16 Stunden bei 20 - 25 °C gerührt. Nach beendeter Reaktion wird im Vakuum das Lösungsmittel abgezogen und der verbleibende ölige Rückstand mit 1 l Diethylether verrührt. Man saugt das ausgeschiedene weiße Pulver ab, wäscht portionsweise mit 1 l Diethylether nach und trocknet das Produkt bei 40 °C im Vakuum.
Ausbeute: 14,31 g (83 %), weißes Pulver.
Wassergehalt: 1,41 %
Dimethylformamidgehalt: 0,8 %

| Analyse (nach Korrektur der Lösungsmittelanteile): | | | |
|---|---|---|---|
| Berechnet:<br>Gefunden: | C 56,43<br>C 56,25 | H 8,77<br>H 8,89 | N 9,75<br>N 9,48 |

3,6-Bis(carboxymethyl)-9-(N-undecylcarbamoylmethyl)-3,6,9-triazaundecandisäure

10 g (17,4 mmol) des in Beispiel 10a) beschriebenen Ethylesters werden in 200 ml 2n Natriumhydroxydlösung gelöst und 2 Stunden bei Raumtemperatur gerührt. Nach beendeter Reaktion wird auf 5 °C gekühlt und solange konzentrierte Salzsäure zugesetzt, bis ein pH-Wert von 2,15 erreicht wird. Das ausgeschiedene weiße Pulver wird abgesaugt und fünfmal mit 50 ml Eiswasser, fünfmal mit Diethylether/Ethanol (8:2), fünfmal mit 50 ml Diethylether und fünfmal mit 50 ml n-Pentan gewaschen.
Ausbeute: 8,25 g (86,7 %) weißes Pulver.
Wassergehalt: 2,12 %

Dimethylformamidgehalt: <0,05 %.

| Analyse (nach Korrektur des Wassergehalts): | | | |
|---|---|---|---|
| Berechnet: | C 54,93 | H 8,48 | N 10,25 |
| Gefunden: | C 54,78 | H 8,67 | N 9,98 |

c) Gadolinium-Komplex der 3,6-Bis(carboxymethyl)-9-(N-undecylcarbamoylmethyl)-3,6,9-triazaundecandisäure

5,47 g (10 mmol) der nach Beispiel 10b) erhaltenen Komplexbildnersäure werden in 500 ml Wasser mit 1,81 g (5 mmol) Gadoliniumoxid 4 Stunden bei 80 °C gerührt, wobei eine fast klare Lösung entsteht. Man filtriert und unterwirft das Filtrat einer Gefriertrocknung.
Ausbeute: 6,35 g (90,6 %), weißes Pulver
Wassergehalt: 4,2 %

| Analyse (nach Korrektur des Wassergehalts) | | | | |
|---|---|---|---|---|
| Berechnet: | C 42,84 | H 6,18 | N 7,99 | Gd 22,44 |
| Gefunden: | C 42,91 | H 6,25 | N 7,87 | Gd 22,40 |

d) Mono-N-Methylglucaminsalz des Gadolinium-Komplexes der 3,6-Bis(carboxymethyl)-9-(N-undecylcarbamoylmethyl)-3,6,9-triazaundecandisäure

2 g des nach Beispiel 10c) erhaltenen Gadolinium-Komplexes werden in 35 ml Wasser gelöst, mit einem Equivalent N-Methylglucamin versetzt, filtriert und die Lösung im Vakuum eingedampft.
Ausbeute: 2,25 g (88 %), weißes Pulver.
Wassergehalt: 3,75 %

| Analyse (nach Korrektur des Wassergehalts): | | | | |
|---|---|---|---|---|
| Berechnet: | C 42,89 | H 6,75 | N 7,82 | Gd 17,55 |
| Gefunden: | C 42,73 | H 6,89 | N 7,69 | Gd 17,48 |

**Beispiel 11**

a) 6-Carboxymethyl-3-ethoxycarbonylmethyl-9-(1-hexadecylcarbamoylmethyl)-3,6,9-triazaundecandisäure

28,20 g (70 mmol) $N^3$-(2,6-Dioxomorpholinoethyl)-$N^6$-(ethoxycarbonylmethyl)-3,6-diazaoctandisäure werden in 320 ml Dimethylformamid bei 0 °C mit 28,8 ml (350 mmol) Triethylamin und 16,90 g (70 mmol) 1-Hexadecylamin versetzt und anschließend 24 Stunden bei 20 - 25 °C gerührt. Man engt dann im Vakuum ein und rührt den Rückstand in der Siedehitze mit 1 l Methyl-tert.-butylether. Nach Abkühlen auf +10 °C saugt man ab, trocknet den Rückstand bei 45 °C im Vakuum, nimmt in 400 ml Wasser auf und gibt die Lösung über eine Säule mit 300 ml Ionenaustauscher IR-120 ($H^+$-Form) wäscht die Säule noch mit 0,5 l Wasser und engt die vereinigten Eluate auf ca. 300 ml im Vakuum ein und isoliert die Titelverbindung durch Gefriertrocknung. Man erhält 36,5 g als weißes Pulver.

Wassergehalt: 1,70 %
Dimethylformamidgehalt: 0,7 %

| Analyse (nach Korrektur der Lösungsmittelanteile): | | | |
|---|---|---|---|
| Berechnet: | C 59,60 | H 9,38 | N 8,69 |
| Gefunden: | C 59,42 | H 9,49 | N 8,85 |

b) 3,6-Bis(carboxymethyl)-9-(1-hexadecylcarbamoylmethyl)-3,6,9-triazaundecandisäure

6,45 g (10 mmol) des in Beispiel 11a) beschriebenen Ethylesters werden in 100 ml Wasser und 9,1 ml 11N Natronlauge gelöst und 2 Stunden bei Raumtemperatur belassen. Man versetzt unter Rühren mit 65 ml Ionenaustauscher Amberlite IR 120 ($H^+$-Form), wobei sich ein pH-Wert von 2,5 einstellt. Man filtriert und unterzieht die Lösung einer Gefriertrocknung.
Man erhält 5,30 g der Titelverbindung als weißes Pulver.
Wassergehalt: 3,2 %
Dimethylformamidgehalt: <0,05 %.

| Analyse (nach Korrektur des Wassergehalts): | | | |
|---|---|---|---|
| Berechnet: | C 58,42 | H 9,15 | N 9,08 |
| Gefunden: | C 58,59 | H 9,44 | N 8,95 |

c) Monomegluminsalz des Gadolinium-Komplexes der 3,6-Bis(carboxymethyl)-9-(1-hexadecylcarbamoylmethyl)-3,6,9-triazaundecandisäure

4,93 g (8 mmol) der nach Beispiel 11b) erhaltenen Komplexbildnersäure werden in 170 ml Wasser mit 1,45 g (4 mmol) Gadoliniumoxid und 1,56 g (8 mmol) N-Methylglucamin 2 Stunden bei 80 - 85 °C gerührt. Die fast klare Lösung wird filtriert und gefriergetrocknet.
Ausbeute: 7,49 g weißes Pulver.
Wassergehalt: 2,8 %

| Analyse (nach Korrektur des Wassergehalts): | | | | |
|---|---|---|---|---|
| Berechnet: | C 45,99 | H 7,30 | N 7,25 | Gd 16,27 |
| Gefunden: | C 46,21 | H 7,55 | N 7,08 | Gd 16,18 |

Auf dem gleichen Weg wie in Beispiel 11 beschrieben läßt sich auch das Monomegluminsalz des Europium-Komplexes der 3,6-Bis(carboxymethyl)-9-(1-hexadecylcarbamoylmethyl)-3,6,9-triazaundecandisäure herstellen.

**Beispiel 12**

Herstellung einer Lösung vom Megluminsalz des Gadolinium-Komplexes des 3,6,9-Tris(carboxymethyl)-3,6,9-triazaundecandisäure-undecylmonoamids

448,57 g (0,5 Mol) der in Beispiel 10 beschriebenen Verbindung werden in 600 ml Wasser pro injectione (p.i.) unter Erwärmen gelöst. Nach Zugabe von 4,92 g (10 mmol) Monohydrat des Calciumtrinatriumsalzes der DTPA, $CaNa_3DTPA$ füllt man mit Wasser p.i. auf 1000 ml auf. Die Lösung wird ultrafiltriert, in Flaschen abgefüllt und hitzesterilisiert und ist für die parenterale Anwendung gebrauchsfertig.

Herstellung einer pulverförmigen Darreichungsform

89,61 g (0,1 Mol) des in Beispiel 10 beschriebenen Megluminsalzes werden mit 25 g Saccharose und 5 g Pluronic(R) F 68 und 10 mg Himbeeraromastoff fein vermahlen. Das Pulver wird in Beutel abgefüllt und ist für die orale Anwendung gebrauchsfertig.

## Ansprüche

1.) Physiologisch verträgliche Komplexverbindungen der allgemeinen Formel I

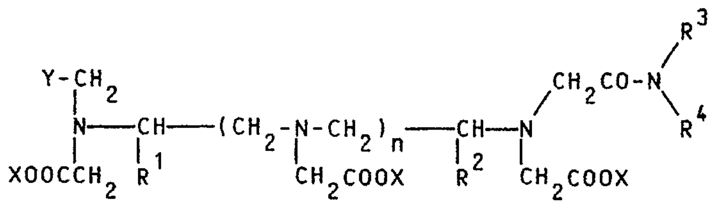

(I)

worin
n die Ziffern 0, 1 oder 2,
$R^1$ und $R^2$ Wasserstoffatome, niedere Alkylgruppen, Phenylgruppen, Benzylgruppen oder, wenn n für die Ziffer 0 steht, auch gemeinsam eine Trimethylen- oder eine Tetramethylengruppe,
$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, wenigstens eine $R^3$-Gruppe, eine Cycloalkyl- oder eine gegebenenfalls durch eine oder mehrere Di-$C_1$-$C_6$-alkanylamino- oder durch eine oder mehrere $C_1$-$C_6$-Alkoxygruppen substituierte Aryl- oder Aralkylgruppe,
$R^4$ ein Wasserstoffatom, ein gesättigter, ungesättigter, gerad- oder verzweigtkettiger oder cyclischer Kohlenwasserstoffrest mit bis zu 16 C-Atomen, oder
$R^3$ und $R^4$ gemeinsam einen gesättigten oder ungesättigten gegebenenfalls durch einen oder mehrere $C_1$-$C_6$-Alkyl, $C_1$-$C_5$-Hydroxyalkyl-, einen gegebenenfalls hydroxylierten oder $C_1$-$C_6$-alkoxylierten $C_2$-$C_6$-Acyl-, Hydroxy-, Carbamoyl-, Carbamoyl-substituierten $C_1$-$C_6$-Alkyl-, am Carbamoyl-Stickstoff durch einen oder zwei $C_1$-$C_6$-Alkylrest(e) - die auch einen gegebenenfalls ein Sauerstoffatom enthaltenen Ring bilden können - substituierten Carbamoyl- oder einen $C_1$-$C_6$-Acylamino- oder $C_1$-$C_6$-Alkylaminorest substituierten 5- oder 6-Ring, der gegebenenfalls ein weiteres Stickstoff-, Sauerstoff-, Schwefelatom oder eine Carbonylgruppe enthält,
X ein Wasserstoffatom und/oder ein Metallionenäquivalent, mindestens eines Elements der Ordungszahlen 21-29, 42, 44, oder 58-70,
Y eine

COOX- oder 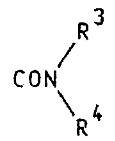 -Gruppe

bedeuten, mit der Maßgabe, daß mindestens zwei der Substituenten X für ein Metallionenäquivalent stehen, sowie deren Salze mit organischen und/oder anorganischen Basen zur Anwendung in der organspezifischen NMR-Diagnostik und bei Patienten mit Niereninsuffizienz.
2.) Physiologisch verträgliche Komplexverbindungen gemäß Anspruch 1, dadurch gekennzeichnet, daß
n die Ziffer 1,
$R^1$ und $R^2$ Wasserstoffatome

$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, wenigstens eine $R^3$-Gruppe, eine Cycloalkyl- oder eine gegebenenfalls durch eine oder mehrere Di-$C_1$-$C_6$-alkanylamino- oder durch eine oder mehrere $C_1$-$C_6$-Alkoxygruppen substituierte Aryl- oder Aralkylgruppe,
$R^4$ ein Wasserstoffatom, ein gesättigter, ungesättigter, gerad- oder verzweigtkettiger oder cyclischer Kohlenwasserstoffrest mit bis zu 16 C-Atomen,
X und Y die in Anspruch 1 angegebenen Reste bedeuten, zur Anwendung in der organspezifischen NMR-Diagnostik und bei Patienten mit Niereninsuffizienz.

3.) Physiologisch verträgliche Komplexverbindungen gemäß Anspruch 1, dadurch gekennzeichnet, daß Y für eine COOX-Gruppe steht, zur Anwendung in der organspezifischen NMR-Diagnostik und bei Patienten mit Niereninsuffizienz.

4.) Physiologisch verträgliche Komplexverbindungen gemäß Anspruch 1, dadurch gekennzeichnet, daß Y für eine

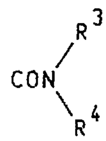 -Gruppe

steht, zur Anwendung in der organspezifischen NMR-Diagnostik und bei Patienten mit Niereninsuffizienz.

5.) Physiologisch verträgliche Komplexverbindungen gemäß Anspruch 2, dadurch gekennzeichnet, daß Y für eine COOX-Gruppe steht, zur Anwendung in der organspezifischen NMR-Diagnostik und bei Patienten mit Niereninsuffizienz.

6.) Physiologisch verträgliche Komplexverbindungen gemäß Anspruch 2, dadurch gekennzeichnet, daß Y für eine

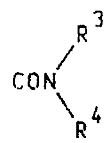 -Gruppe

steht, zur Anwendung in der organspezifischen NMR-Diagnostik und bei Patienten mit Niereninsuffizienz.

7.) Verwendung von mindestens einer physiologisch verträglichen Komplexverbindung gemäß Anspruch 1 für die Herstellung von Mitteln für die organspezifische NMR-Diagnostik und bei Patienten mit Niereninsuffizienz.

8.) Verwendung von mindestens einer physiologisch verträglichen Komplexverbindung gemäß Anspruch 2 für die Herstellung von Mitteln für die organspezifische NMR-Diagnostik und bei Patienten mit Niereninsuffizienz.

9.) Verwendung von mindestens einer physiologisch verträglichen Komplexverbindung gemäß Anspruch 1, dadurch gekennzeichnet, daß Y für eine COOX-Gruppe steht, für die Herstellung von Mitteln für die organspezifische NMR-Diagnostik und bei Patienten mit Niereninsuffizienz.

10.) Verwendung von mindestens einer physiologisch verträglichen Komplexverbindung gemäß Anspruch 1, dadurch gekennzeichnet, daß Y für eine

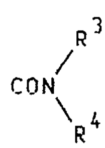 -Gruppe

steht, für die Herstellung von Mitteln für die organspezifische NMR-Diagnostik und bei Patienten mit Niereninsuffizienz.

11.) Verwendung von mindestens einer physiologisch verträglichen Komplexverbindung gemäß Anspruch 2, dadurch gekennzeichnet, daß Y für eine COOX-Gruppe steht, für die Herstellung von Mitteln für die organspezifische NMR-Diagnostik und bei Patienten mit Niereninsuffizienz.

12.) Verwendung von mindestens einer physiologisch verträglichen Komplexverbindung gemäß Anspruch 2, dadurch gekennzeichnet, daß Y für eine

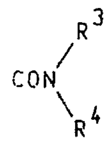 -Gruppe

steht, für die Herstellung von Mitteln für die organspezifische NMR-Diagnostik und bei Patienten mit Niereninsuffizienz.

13.) Gadoliniumkomplex des 3,6,9-Tris(carboxymethyl)-3,6,9-triazaundecandisäure-phenylmonoamids,
Gadoliniumkomplex des 3,6,9-Tris(carboxymethyl)-3,6,9-triazaundecandisäure-(N,N'-diphenyl)-diamids,
Gadoliniumkomplex des 3,6,9-Tris(carboxymethyl)-3,6,9-triazaundecandisäure(N,N'-dibenzyl)-diamids,
Gadoliniumkomplex des 3,6,9-Tris(carboxymethyl)-3,6,9-triazaundecandisäure-benzylmonoamids,
Dysprosium-(III)-Komplex von 3,6,9-Tris(caraboxymethyl)-3,6,9-triazaundecandisäure-benzylmonoamid,
Gadoliniumkomplex des 3,6,9-Tris(carboxymethyl)-3,6,9-triazaundecandisäure-tert.-butylmonoamids,
Gadoliniumkomplex der 3,6-Bis(carboxymethyl)-9-(4-methoxybenzylcarbamoylmethyl)-3,6,9-triazaundecandisäure,
Gadoliniumkomplex der 3,6-Bis(carboxymethyl)-9-(N-undecylcarbamoylmethyl)-3,6,9-triazaundecandisäure,
Gadoliniumkomplex der 3,6-Bis(carboxymethyl)-9-(1-hexadecylcarbamoylmethyl)-3,6,9-triazaundecandisäure
sowie deren Salze mit organischen und/oder anorganischen Basen.